# EUROPÄISCHE PATENTSCHRIFT

(11) **EP 1 318 138 B1**
(45) Veröffentlichungstag und Bekanntmachung des Hinweises auf die Patenterteilung: **29.09.2004**
(21) Anmeldenummer: 02025292.0
(22) Anmeldetag: 13.11.2002
(51) Int. Cl.: C07C 67/317

(54) **Verfahren zur Herstellung von Dihydroxycarbonsäureestern in Abwesenheit von Lösungsmitteln**
Process for the preparation of dihydroxycarboxylic acid esters in the absence of solvents
Procédé de préparation d'esters d'acides dihydroxycarboxyliques en absence de solvants

(30) Priorität: 07.12.2001 DE 10160149
(43) Veröffentlichungstag der Anmeldung: 11.06.2003
(73) Patentinhaber: BASF AKTIENGESELLSCHAFT, 67056 Ludwigshafen (DE)
(72) Erfinder: Klatt, Martin Jochen, Dr., 67098 Bad Dürkheim (DE); Niebel, Markus, 68163 Mannheim (DE); Erhardt, Melanie, 67245 Lambsheim (DE)

(56) Entgegenhaltungen:
- EP-A- 0 487 986
- EP-A- 0 863 125
- BRINGMANN G ET AL: "A SHORT AND PRODUCTIVE SYNTHESIS OF (R)-ALPHA-LIPOIC ACID" ZEITSCHRIFT FUR NATURFORSCHUNG, TEIL B: ANORGANISCHE CHEMIE, ORGANISCHE CHEMIE, VERLAG DER ZEITSCHRIFT FUR NATURFORSCHUNG. TUBINGEN, DE, Bd. 54B, 1999, Seiten 655-661, XP001063331 ISSN: 0932-0776

## Beschreibung

Die vorliegende Erfindung betrifft ein Verfahren zur Herstellung von Dihydroxycarbonsäureestern sowie ein Gesamtverfahren zur Herstellung von R-(+)-α-Liponsäure.

Dihydroxycarbonsäureester stellen wertvolle Zwischenprodukte und Synthesebausteine in der organischen Chemie dar. Insbesondere dienen (6S)-6,8-Dihydroxyoctansäureester als Zwischenprodukte für die Synthese von enantiomerenreiner R-(+)-α-Liponsäure.

Aus EP 487 986 ist bekannt, (6S)-6,8-Dihydroxyoctansäureester durch Reduktion der entsprechenden (3S)-3-Hydroxyoctandisäurediester mit komplexen Hydriden in Gegenwart von aprotischen Lösungsmitteln herzustellen.

Mit diesem Verfahren werden zwar gute aber noch verbesserungsbedürftige Ausbeuten erreicht. Ferner weist das Verfahren die Nachteile auf, daß Lösungsmittel und größere Mengen an komplexen Hydriden verwendet werden müssen.

Der Erfindung lag somit die Aufgabe zu Grunde, ein Verfahren zur Herstellung von Dihydroxycarbonsäureestern zur Verfügung zu stellen, das die Nachteile des Standes der Technik nicht aufweist und die Dihydroxycarbonsäureester lösungsmittelfrei und in besseren Ausbeuten liefert.

Demgemäß wurde gefunden, ein Verfahren zur Herstellung von Dihydroxycarbonsäureestern der Formel I, 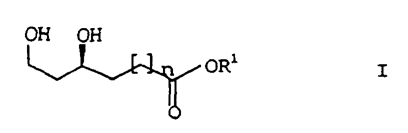 wobei
- n: 1, 2, 3, 4, 5, 6 oder 7 und
- R¹: einen gegebenenfalls substituierten C₁-C₂₀-Alkyl-, C₂-C₂₀-Alkenyl-, C₂-C₂₀ Alkinyl-, C₃-C₈-Cycloalkyl-, Aralkyl-, Aryl-, Hetarylalkyl- oder Hetarylrest bedeuten,
durch Umsetzen von Hydroxycarbondisäurediester der Formel II, 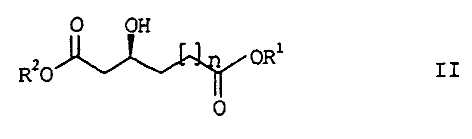 wobei
- R²: einen von R¹ unabhängigen Rest R¹ bedeutet,
mit komplexen Hydriden in Abwesenheit eines Lösungsmittels.

Der Index n, der die Anzahl der -CH₂-Reste darstellt, bedeutet 1, 2, 3, 4, 5, 6 oder 7, vorzugsweise 3. In einer bevorzugten Ausführungsform des Verfahrens werden demnach erfindungsgemäß Dihydroxyoctansäureester hergestellt.

Die Reste R¹ und R² können verschieden oder identisch sein. Die Reste R¹ und R² bedeuten demnach unabhängig voneinander einen gegebenenfalls substituierten C₁-C₂₀-Alkylrest, vorzugsweise C₁-C₁₂-Alkylrest, besonders bevorzugt C₁-C₄-Alkylrest, einen gegebenenfalls substituierten C₂-C₂₀-Alkenylrest, vorzugsweise C₂-C₁₂-Alkenylrest, besonders bevorzugt C₁-C₄-Alkenylrest, einen gegebenenfalls substituierten C₂-C₂₀ Alkinylrest, vorzugsweise C₂-C₁₂-Alkinylrest, besonders bevorzugt C₁-C₄-Alkinylrest, einen gegebenenfalls substituierten C₃-C₈-Cycloalkylrest, einen gegebenenfalls substituierten Aralkylrest, einen gegebenenfalls substituierten Arylrest, einen gegebenenfalls substituierten Hydroxyalkylrest oder einen gegebenenfalls substituierten Hetarylrest.

Unter gegebenenfalls substituierten Resten werden erfindungsgemäß die entsprechenden unsubstituierten und substituierten Reste verstanden. Für alle substituierten Reste der vorliegenden Erfindung kommen, wenn die Substituenten nicht näher spezifiziert sind, unabhängig voneinander bis zu 5 Substituenten, beispielsweise ausgewählt aus der folgenden Gruppe in Frage:

Halogen, insbesondere F oder Cl, gegebenenfalls substituierte C₁-C₁₂-Alkylreste, insbesondere C₁-C₄-Alkylreste, wie beispielsweise Methyl, CF₃, C₂F₅ oder CH₂F oder C₁-C₁₂-Alkoxyreste, insbesondere C₁-C₄-Alkoxyreste.

C₁-C₁₂-Alkylreste für R₁ und R₂ sind unabhängig voneinander beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, Hexyl, 1-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, Heptyl, Octyl, Nonyl, Decyl, Undecyl oder Dodecyl, vorzugsweise verzweigte oder unverzweigte C₁-C₄-Alkylreste wie beispielsweise Methyl, Ethyl, Propyl, 1-Methylethyl, Butyl, 1-Methylpropyl, 2-Methylpropyl oder 1,1-Dimethylethyl, besonders bevorzugt Methyl.

Unter einem C₂-C₁₂-Alkenylrest werden für R₁ und R₂ unabhängig voneinander beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-entenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-2-propenyl sowie die entsprechenden Heptenyle, Octenyle, Nonenyle, Decenyle, Undecenyle und Dodecenyle verstanden.

Unter einem C₂-C₁₂-Alkinylrest werden für R₁ und R₂ unabhängig voneinander beispielsweise Ethinyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise Ethinyl, 2-Propinyl, 2-Butinyl, 1-Methyl-2-propinyl oder 1-Methyl-2-butinyl, sowie die entsprechenden Heptinyle, Octinyle, Noninyle, Decinyle, Undecinyle und Dodecinyle verstanden.

Unter einem C₃-C₈-Cycloalkylrest werden für R¹ und R² unabhängig voneinader beispielsweise Cyclopropyl, Cyclobutyl, Cyclopentyl, Cyclohexyl, Cycloheptyl oder Cyclooctyl verstanden.

Bevorzugte, gegebenenfalls substituierte Arylreste für R₁ und R₂ sind unabhängig voneinander gegebenenfalls substituiertes Phenyl, 1-Naphthyl oder 2-Naphthyl.

Bevorzugte, gegebenenfalls substituierte Arylalkylreste für R₁ und R₂ sind unabhängig voneinander gegebenenfalls substituiertes Benzyl oder Ethylenphenyl (Homobenzyl).

Unter Hetarylresten für R¹ und R² werden unabhängig voneinander beispielsweise Reste wie 2-Pyridyl, 3-Pyridyl, 4-Pyridyl, 2-Furyl, 3-Furyl, 2-Pyrrolyl, 3-Pyrrolyl, 2-Thienyl, 3-Thienyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl verstanden.

Unter substituierten Hetarylresten für R¹ und R² werden unabhängig voneinander auch anellierte Derivate der vorstehend erwähnten Hetarylreste verstanden, wie beispielsweise Indazol, Indol, Benzothiophen, Benzofuran, Indolin, Benzimidazol, Benzthiazol, Benzoxazol, Chinolin, 2,3-Dihydro-1-benzofuran, Furo[2,3]pyridin, Furo[3,2]pyridin oder Isochinolin.

Unter Hetarylalkylresten werden für R¹ und R² unabhängig voneinander Reste verstanden, die sich beispielsweise aus C₁-C₆-Alkylenresten und aus den vorstehend beschriebenen Hetarylresten zusammensetzen, wie beispielsweise die Reste -CH₂-2-Pyridyl, -CH₂-3-Pyridyl, -CH₂-4-Pyridyl, -CH₂-2-Thienyl, -CH₂-3-Thienyl, - CH₂-2-Thiazolyl, -CH₂-4-Thiazolyl, CH₂-5-Thiazolyl, -CH₂-CH₂-2-Pyridyl, -CH₂-CH₂-3-Pyridyl, -CH₂-CH₂-4-Pyridyl, - CH₂-CH₂-2-Thienyl, -CH₂-CH₂-3-Thienyl, -CH₂-CH₂-2-Thiazolyl, -CH₂-CH₂-4-Thiazolyl, oder -CH₂-CH₂-5-Thiazolyl.

Bevorzugte Reste für R¹ und R² sind unabhängig voneinander nicht substituierte Reste. Besonders bevorzugte Reste für R¹ und R² sind unabhängig voneinander die vorstehend beschriebenen C₁-C₁₂-Alkylreste, insbesondere C₁-C₄-Alkylreste, insbesondere Methyl.

In einer besonders bevorzugten Ausführungsform sind die Reste R¹ und R² identisch.

Die Ausgangsverbindungen des erfindungsgemäßen Verfahrens sind die Hydroxycarbondisäurediester der Formel II. Die Herstellung dieser Ausgangsverbindungen ist an sich bekannt und beispielsweise in EP 487 986 und in der darin zitierten Literatur beschrieben. Bevorzugte Hydroxycarbondisäurediester der Formel II als Ausgangsverbindungen setzen sich aus den vorstehend beschriebenen bevorzugten Resten R¹ und R² und dem bevorzugten Index n zusammen.

Besonders bevorzugte Hydroxycarbondisäurediester der Formel II als Ausgangsverbindung sind:
(3S)-3-Hydroxyoctandisäuredimethylester,
(3S)-1-Ethyl-3-hydroxy-8-methyl-octandioat,
(3S)-3-Hydroxy-8-methyl-1-propyl-octandioat,
(3S)-3-Hydroxy-8-methyl-1-iso-propyl-octandioat,
(3S)-1-Butyl-3-hydroxy-8-methyl-octandioat,
(3S)-3-Hydroxy-1-sec.-butyl-8-methyl-octandioat,
(3S)-3-Hydroxy-8-methyl-1-tert.-butyl-octandioat,
(3S)-3-Hydroxy-8-methyl-1-octyl-octandioat,
(3S)-3-Hydroxy-8-methyl-1-phenyl-octandioat und
(3S)-1-(2-Ethylhexyl)-3-hydroxy-8-methyl-octandioat

Eine besonders bevorzugte Ausgangsverbindung ist (3S)-3-Hydroxyoctandisäuredimethylester.

Die erfindungsgemäße Herstellung von Dihydroxycarbonsäureestern der Formel I als Produktverbindungen erfolgt durch Umsetzen von Hydroxycarbondisäurediester der Formel II als Ausgangsverbindungen mit komplexen Hydriden und damit durch Reduktion der Hydroxycarbondisäurediester der Formel II als Ausgangsverbindungen in Abwesenheit eines Lösungsmittels.

"In Abwesenheit eines Lösungsmittels" bedeutet erfindungsgemäß, daß während der erfindungsgemäßen Umsetzung kein Lösungsmittel zugegen ist. Unter Lösungsmittel werden, in an sich bekannter Weise, inerte flüssige Verbindungen verstanden, die nicht als Reaktanden an der Reaktion teilnehmen und die die Verbindungen der Reaktionsmischung lösen oder suspendieren können.

Insbesondere werden aprotische Lösungsmittel als Lösungsmittel verstanden, wie beispielsweise aliphatische und aromatische Kohlenwasserstoffe wie beispielsweise Hexan, Cyclohexan, Toluol, Benzol und Xylol sowie Ether wie beispielsweise Dioxan, Diethylether und Tetrahydrofuran.

Die Hydroxycarbondisäurediester der Formel II, die komplexen Hydride oder die während der Reaktion freigesetzten Alkohole werden nicht als Lösungsmittel verstanden.

Nach der Umsetzung und während der Aufarbeitung ist es vorteilhaft Lösungsmittel zu verwenden, um Nebenprodukte abzutrennen und die gewünschten Produkte zu isolieren.

Das erfindungsgemäße Verfahren kann prinzipiell in allen Reaktoren durchgeführt werden, die eine ausreichende Durchmischung der Reaktionsmischung während der Umsetzung gewährleisten.

Besonders bevorzugte Reaktoren für das erfindungsgemäße Verfahren sind Knetapparaturen, die in an sich bekannter Weise auch Kneter genannt werden.

Das erfindungsgemäße Verfahren kann diskontinuierlich oder kontinuierlich durchgeführt werden.

In einer bevorzugten Ausführungsform erfolgt die Durchführung des erfindungsgemäßen Verfahrens diskontinuierlich.

Für die diskontinuierlichen Ausführungsform des Verfahrens ist die Verwendung diskontinuierlicher Knetapparaturen als Reaktoren, wie beispielsweise die Verwendung von Einarmkneter, Schaufelkneter, Planetenkneter oder Kreiselmischer besonders bevorzugt.

In einer weiteren bevorzugten Ausführungsform erfolgt die Durchführung des erfindungsgemäßem Verfahrens kontinuierlich.

Vorzugsweise wird das kontinuierliche Verfahren dabei so durchgeführt, daß man die Hydroxycarbondisäurediester der Formel II und die komplexen Hydride kontinuierlich einem Reaktor zuführt und die Dihydroxycarbonsäureestern der Formel I kontinuierlich aus dem Reaktor ausführt.

Für die kontinuierliche Ausführungsform der Verfahrens ist die Verwendung kontinuierlicher Knetapparaturen als Reaktoren bevorzugt. Bevorzugte kontinuierliche Knetapparaturen sind Extruder, Walzenkneter, wie beispielsweise Ein- und Mehrfachwalzwerke und Walzenstühle sowie Schneckenkneter, wie beispielsweise Einschnekkenkneter mit und ohne axialer Schneckenbewegung und Mehrschnekkenkneter. Besonders bevorzugte kontinuierliche Knetapparaturen sind Extruder und Schneckenkneter.

Bevorzugte komplexe Hydride sind Borhydride, insbesondere Ammonium-, Lithium-, Kalium- und Natriumborhydrid, sowie alkyl- und alkoxysubstituierte Borhydride wie beispielsweise Lithiumtriethylborhydrid und Natriumtrimethoxyborhydrid. Besonders bevorzugtes komplexes Hydrid im erfindungsgemäßen Verfahren ist Natriumborhydrid.

Das Molverhältnis der komplexen Hydride zum Hydroxycarbondisäurediester der Formel II ist nicht kritisch und beträgt typischerweise 0,5:1 bis 3:1, vorzugsweise 0,5:1 bis 1:1, besonders bevorzugt 0,6:1 bis 0,8:1.

Die Temperatur bei der das erfindungsgemäße Verfahren durchgeführt wird ist nicht kritisch und beträgt typischerweise 0 bis 150°C, vorzugsweise 10 bis 60°C. Das erfindungsgemäße Verfahren erfolgt typischerweise bei Normaldruck, kann aber auch bei verminderten oder leicht erhöhtem Druck durchgeführt werden, vorzugsweise bei 0,1 bis 10 bar. Die Reaktionszeiten sind nicht kritisch und betragen typischerweise 0,1 bis 5 Stunden, insbesondere 0,5 bis 1 Stunde.

Die Isolierung der Dihydroxycarbonsäureester der Formel I erfolgt in an sich bekannter Weise, beispielsweise durch Aufarbeitung des Reaktionsgemisches durch Hydrolyse, Extraktion und Trocknung.

Das erfindungsgemäße Verfahren weist den Vorteil auf, daß die Dihydroxycarbonsäureester der Formel I ohne Verwendung von Lösungsmitteln, in hohen Ausbeuten, schnellen Reaktionszeiten und unter Verwendung geringerer Mengen komplexer Hydride hergestellt werden können.

Die Erfindung betrifft weiterhin ein Gesamtverfahren zur Herstellung von R-(+)-α-Liponsäure unter Verwendung des erfindungsgemäßen Verfahrens als Zwischenschritt.

Daher betrifft die Erfindung ein Verfahren zur Herstellung von R-(+)-α-Liponsäure der Formel IV 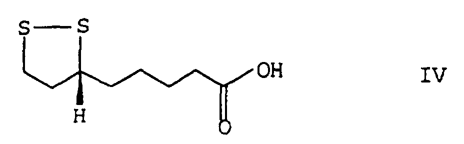 dadurch gekennzeichnet, daß man Dihydroxycarbonsäureestern der Formel I, wobei n gleich 3 bedeutet, durch Umsetzung der entsprechenden Hydroxycarbondisäurediester der Formel II mit komplexen Hydriden in Abwesenheit eines Lösungsmittels herstellt und man diese Dihydroxycarbonsäureestern der Formel I in an sich bekannter Weise
a) in organischer Lösung mit einem Sulfonsäurechlorid und einer tertiären Stickstoffbase in den Bissulfonsäureester von I überführt,
b) diese Bissulfonsäureester in einem polaren Lösungsmittel mit Schwefel und einem Alkalimetalldisulfid zum R-α-Liponsäureester umsetzt und
c) dieser Ester in die R-(+)-α-Liponsäure der Formel IV überführt.

Das folgende Beispiel erläutert die Erfindung.

### Beispiel 1

45,3 g (0,2 mol) enantiomerenreiner (3S)-3-Hydroxyoctandisäuredimethylester wurden in einem Haake-Kneter bei 30 bis 40°C mit 5,2 g Natriumborhydrid (0,14 mol, 0,7 Äquivalente) vermengt. Das Reaktionsgemisch wurde geknetet bis die Reaktion beendet war (DC-Kontrolle, 40 min). Im Anschluss wurde der intermediäre Borsäuremethylester in Methanol aufgenommen und unter sauren Bedingungen hydrolisiert. Der gewünschte (6S)-6,8-Dihydroxyoctansäuremethylester wurde mit Essigester extrahiert. Nach Trocknen der organischen Phase wurde diese auf 0°C abgekühlt und das auskristallisierte Produkt abfiltriert. Man erhält 34,5 g (6S)-6,8-Dihydroxyoctansäuremethylester in Form eines weißen Kristallisats (90 % Ausbeute).

## Patentansprüche

1. Verfahren zur Herstellung von Dihydroxycarbonsäureestern der Formel I, 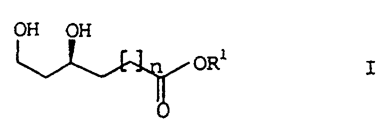 wobei
n 1, 2, 3, 4, 5, 6 oder 7 und
R¹ einen gegebenenfalls substituierten C₁-C₂₀-Alkyl-, C₂-C₂₀-Alkenyl-, C₂-C₂₀ Alkinyl-, C₃-C₈-Cycloalkyl-, Aralkyl-, Aryl-, Hetarylalkyl- oder Hetarylrest bedeuten
durch Umsetzen von Hydroxycarbondisäurediester der Formel II, 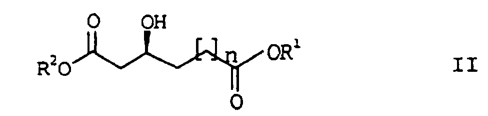 wobei
R² einen von R¹ unabhängigen Rest R¹ bedeutet,
mit komplexen Hydriden in Abwesenheit eines Lösungsmittels.

2. Verfahren nach Anspruch 1, **dadurch gekennzeichnet, daß** man die Reaktion in einer Knetapparatur durchführt.

3. Verfahren nach Anspruch 1 oder 2 **dadurch gekennzeichnet, daß** man die Reaktion kontinuierlich durchführt.

4. Verfahren nach Anspruch 3, **dadurch gekennzeichnet daß** man die Hydroxycarbondisäurediester der Formel II und die komplexen Hydride kontinuierlich einem Reaktor zuführt und die Dihydroxycarbonsäureestern der Formel I kontinuierlich aus dem Reaktor ausführt.

5. Verfahren nach einem der Ansprüche 1 bis 4, **dadurch gekennzeichnet, daß** man als komplexes Hydrid Natriumborhydrid verwendet.

6. Verfahren zur Herstellung von R-(+)-α-Liponsäure der Formel IV 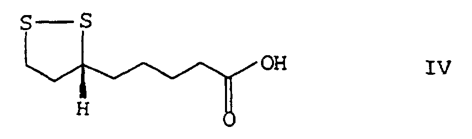 **dadurch gekennzeichnet,**
**daß** man Dihydroxycarbonsäureestern der Formel I, 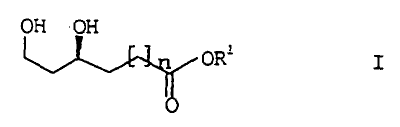 wobei
n 3 und
R¹ einen gegebenenfalls,substituierten C₁-C₂₀-Alkyl-, C₂-C₂₀-Alkenyl-, C₂-C₂₀ Alkinyl-, C₃-C₈-Cycloalkyl-, Aralkyl-, Aryl-, Hetarylalkyl- oder Hetarylrest bedeuten
durch Umsetzung von Hydroxycarbondisäurediester der Formel II, 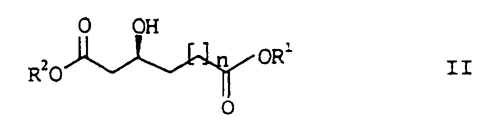 wobei
R² einen von R¹ unabhängigen Rest R¹ bedeutet,
mit komplexen Hydriden in Abwesenheit eines Lösungsmittels herstellt und man diese Dihydroxycarbonsäureester der Formel I
a) in organischer Lösung mit einem Sulfonsäurechlorid und einer tertiären Stickstoffbase in den Bissulfonsäureester von I überführt,
b) diese Bissulfonsäureester in einem polaren Lösungsmittel mit Schwefel und einem Alkalimetalldisulfid zum R-α-Liponsäureester umsetzt und
c) dieser Ester in die R-(+)-α-Liponsäure der Formel IV überführt.

## Claims

1. A process for preparing dihydroxycarboxylic esters of the formula I, 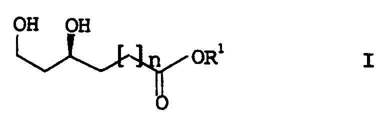 where
n is 1, 2, 3, 4, 5, 6 or 7 and
R¹ is an unsubstituted or substituted C₁-C₂₀-alkyl, C₂-C₂₀-alkenyl, C₂-C₂₀-alkynyl, C₃-C₈-cycloalkyl, aralkyl, aryl, hetarylalkyl or hetaryl radical,
which comprises reacting hydroxycarboxylic diesters of the formula II, 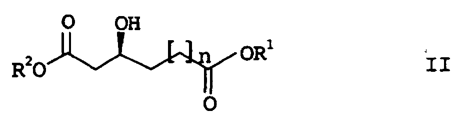 where
R² is a radical R¹ independent of R¹,
with complex hydrides in the absence of a solvent.

2. A process as claimed in claim 1, wherein the reaction is carried out in a kneading apparatus.

3. A process as claimed in claim 1 or 2, wherein the reaction is carried out continuously.

4. A process as claimed in claim 3, wherein the hydroxycarboxylic diesters of the formula II and the complex hydrides are fed continuously to a reactor and the dihydroxycarboxylic esters of the formula I are removed continuously from the reactor.

5. A process as claimed in one of claims 1 to 4, wherein the complex hydride used is sodium borohydride.

6. A process for preparing R-(+)-α-lipoic acid of the formula IV 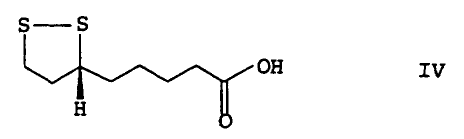 which comprises
preparing dihydroxycarboxylic esters of the formula I, 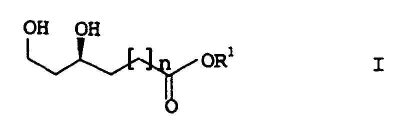 where
n is 3 and
R¹ is an unsubstituted or substituted C₁-C₂₀-alkyl, C₂-C₂₀-alkenyl, C₂-C₂₀-alkynyl, C₃-C₈-cycloalkyl, aralkyl, aryl, hetarylalkyl or hetaryl radical
by reacting hydroxycarboxylic diesters of the formula II, 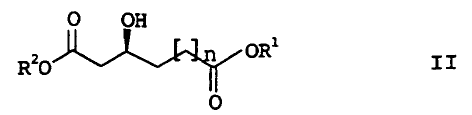 where
R² is a radical R¹ independent of R¹,
with complex hydrides in the absence of a solvent and
a) converting these dihydroxycarboxylic esters of the formula I in organic solution using a sulfonyl chloride and a tertiary nitrogen base into the bissulfonic esters of I,
b) reacting these bissulfonic esters with sulfur and an alkali metal disulfide in a polar solvent to give the R-α-lipoic ester and
c) converting this ester into the R-(+)-α-lipoic acid of the formula IV.

## Revendications

1. Procédé pour la préparation d'esters d'acide dihydroxycarboxylique de formule I, 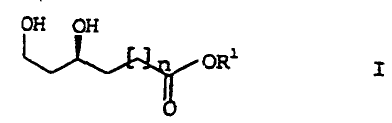 où
nvaut 1, 2, 3, 4, 5, 6 ou 7 et
R¹ désigne un reste alkyle en C₁-C₂₀, alcényle en C₂-C₂₀, alcynyle en C₂-C₂₀, cycloalkyle en C₃-C₈, aralkyle, aryle, hétarylalkyle ou hétaryle, éventuellement substitué,
par réaction de diesters de diacide hydroxycarboxylique de formule II, 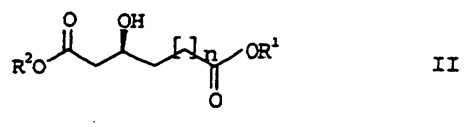 où
R² représente un reste R¹ indépendant de R¹,
avec des hydrures complexes en l'absence de solvant.

2. Procédé selon la revendication 1, **caractérisé par le fait qu'**on effectue la réaction dans un appareil malaxeur. par réaction de diesters de diacide hydroxycarboxylique de formule II, 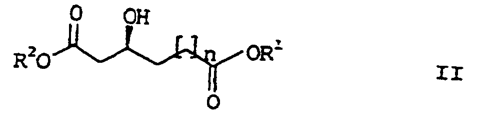 où
R² représente un reste R¹ indépendant de R¹,
avec des hydrures complexes en l'absence de solvant et ces esters d'acide dihydroxycarboxylique de formule I
a) sont transformés en les esters d'acide bissulfonique de I en solution organique avec du chlorure d'acide sulfonique et une base azotée tertiaire,
b) ces esters d'acide bissulfonique sont mis à réagir dans un solvant polaire avec du soufre et un disulfure de métal alcalin pour donner l'ester d'acide R-α-lipoïque et
c) cet ester est converti en l'acide R-α-lipoïque de formule IV.
